# FASCICULE DE BREVET EUROPEEN

(11) **EP 1 488 004 B1**
(45) Date de publication et mention de la délivrance du brevet: **13.09.2006**
(21) Numéro de dépôt: 03727612.8
(22) Date de dépôt: 24.03.2003
(51) Int. Cl.: C12Q 1/68

(54) **PROCEDE DE CONCENTRATION DE MACROMOLECULES OU AGGLOMERATS DE MOLECULES OU DE PARTICULES**
VERFAHREN ZUR AUFKONZENTRIERUNG VON MAKROMOLEKÜLEN ODER AGGLOMERATEN AUS MOLEKÜLEN ODER TEILCHEN
METHOD OF CONCENTRATING MACROMOLECULES OR AGGLOMERATES OF MOLECULES OR PARTICLES

(30) Priorité: 25.03.2002 FR 0203690
(43) Date de publication de la demande: 22.12.2004
(73) Titulaire: COMMISSARIAT A L'ENERGIE ATOMIQUE, 75752 Paris Cédex 15 (FR); CENTRE NATIONAL DE LA RECHERCHE SCIENTIFIQUE (CNRS), 75794 Paris Cedex 16 (FR)
(72) Inventeur: BERTHIER, Jean, F-38240 MEYLAN (FR); DAVOUS, Laurent, F-72510 REQUEIL (FR)
(74) Mandataire: Poulin, Gérard
(86) Numéro de dépôt international: PCT/FR2003/000920
(87) Numéro de publication internationale: WO 2003/080209

(56) Documents cités:
- EP-A- 0 245 985
- WO-A-97/34909
- US-A- 5 024 952
- US-A- 6 020 131
- PATENT ABSTRACTS OF JAPAN vol. 015, no. 096 (C-0812), 7 mars 1991 (1991-03-07) & JP 02 307571 A (FUJI PHOTO FILM CO LTD), 20 décembre 1990 (1990-12-20)

## Description

### DOMAINE TECHNIQUE

La présente invention a trait à un procédé de concentration de macromolécules ou d'agglomérats de molécules ou de particules, en vue d'une éventuelle détection ou d'une extraction spécifique desdites macromolécules ou desdits agglomérats.

### ETAT DE LA TECHNIQUE ANTERIEURE

Actuellement, les techniques de concentration de macromolécules ou d'agglomérats de molécules sont, pour la plupart, destinées au domaine du diagnostic médical, notamment au domaine de la détection de brins d'ADN ou de complexes protéiniques tels que les antigènes ou les prions.

Pour la détection de brins d'ADN, la technique actuelle consiste, le plus souvent, à concentrer des brins d'ADN par amplification dans un milieu liquide, selon la technique communément désignée par l'abréviation PCR (« Polymerase Chain Réaction » ou « Réaction en Chaîne par Polymérase »).

Cette technique consiste à répliquer les brins d'ADN contenus dans un échantillon liquide un grand nombre de fois (jusqu'à 10⁵-10⁶ fois) en injectant de la polymérase dans l'échantillon soumis à une série de cycles thermiques.

Après un nombre de cycles thermiques assez important, en tout cas supérieur à 10, la concentration en ADN est suffisamment importante pour permettre la détection.

Toutefois, cette technique, telle qu'explicitée ci-dessus, nécessite une durée importante, du fait du nombre conséquent de cycles thermiques à reproduire pour avoir une quantité d'ADN suffisante. De plus, cette technique s'accompagne d'un bruit de fond important du fait que la polymérase peut amplifier des segments d'ADN présents dans l'échantillon liquide, qui sont de nature différente des segments d'ADN à détecter.

Afin de pallier ces inconvénients, des méthodes alternatives à la PCR ont été développées. Parmi ces méthodes, on peut citer une méthode ne nécessitant pas d'amplification. Le principe de cette méthode de détection sans amplification repose sur la capture des segments d'ADN cibles aussi peu nombreux soient-ils. Elle consiste à hybrider les segments d'ADN cibles avec des nanobilles paramagnétiques fonctionnalisées de manière à concentrer lesdits segments à la surface de ces billes avant détection. Cette méthode se heurte toutefois au problème de l'adsorption non spécifique, certaines billes paramagnétiques, enrobées de latex, se collant aux parois solides du réacteur, au niveau duquel est mise en oeuvre la méthode, sous l'effet de l'hydrophobie ou des forces électriques. La sensibilité atteinte n'est alors plus celle escomptée.

Le document US A 6 020 131 (K. Ijiro, M. Shimomura) concerne divers procédés d'identification d'un acide nucléique cible présent dans un échantillon liquide basés sur la formation d'une monocouche d'un pigment capable de s'intercaler entre les bases d'un acide nucléique, à l'interface eau-air de l'échantillon liquide. Ledit acide nucléique cible présent dans l'échantillon liquide va se lier au pigment formant la monocouche et ainsi modifier la pression de surface de cette monocouche. La mesure de ces variations de pression reflète la présence de l'acide nucléique cible.

En ce qui concerne la détection de protéines telles que les anticorps ou les antigènes, deux types de tests se distinguent : les tests dits « en phase homogène » et ceux en « phase hétérogène ». Les tests en phase homogène ont lieu en solution. La mesure de la quantité d'anticorps est réalisée directement dans la solution par fixation de cet anticorps sur l'antigène complémentaire, sans qu'il y ait de séparation physique entre les anticorps libres et les anticorps liés à un antigène. Cette distinction entre anticorps libre et anticorps lié est faite grâce à diverses astuces, notamment basées, par exemple, sur un transfert de fluorescence entre deux anticorps (l'antigène étant pris en sandwich entre deux anticorps marqués par exemple). Ces tests présentent l'avantage d'être rapides, et peu coûteux. Ce sont eux qui sont massivement utilisés dans les programmes de criblage haut débit de l'industrie pharmaceutique par exemple. Toutefois, leur sensibilité est limitée, aux environs de 1nM en ce qui concerne les tests basés sur les acides nucléiques. C'est pourquoi l'industrie du diagnostic utilise préférentiellement des tests en phase hétérogène, dans lesquels un anticorps est immobilisé sur un support solide, support qu'il sera alors possible de laver pour éliminer la quasi-totalité des réactifs de marquage. De plus, une amplification du signal est réalisée, en utilisant une enzyme comme molécule de marquage. Ces tests sur support solide sont plus lents et plus onéreux que ceux en phase homogène, mais ils permettent d'atteindre des sensibilités bien plus grandes, aux alentours de 0,1 pM, soit 10 000 fois mieux que les tests en phase homogène. Toutefois, l'utilisation d'un support solide rend ces tests difficiles à mettre en oeuvre.

En ce qui concerne les complexes protéiniques tels que les prions, ceux-ci doivent également, pour être détectables, dans des liquides physiologiques, tels que le sang, subir une phase de concentration. Pour ces molécules, la technique PCR précédemment décrite est inutilisable, parce qu'ils ne contiennent pas de nucléotides. De ce fait, des recherches relatives au domaine des prions ont abouti récemment à la mise en place d'une méthode dite 'méthode d'amplification des protéines anormalement repliées' ou PMCA (pour 'Protein Misfolding Cyclic Amplification').

Les prions, responsables notamment des encéphalopathies spongiformes, sont des complexes ou agglomérats constitués d'une protéine naturelle, la glycoprotéine PrP^{C}, normalement présente à la surface de nombreuses cellules dans l'organisme et d'une protéine infectieuse PrP^{SC}, qui ne se différencie de la glycoprotéine normale PrP^{C} que par sa conformation, qui est anormalement repliée. Les protéines PrP^{SC} sont aptes, d'une part, à s'associer aux protéines PrP^{C} et d'autre part, aptes à induire la transformation de protéines normales en protéines infectieuses. La détection des prions est rendue difficile par le fait qu'ils ne sont présents en quantité notable que dans le cerveau, alors qu'ils se retrouvent à l'état de traces dans le sang.

La technique PMCA permet ainsi de concentrer les prions dans le milieu de prélèvement, c'est-à-dire le sang, afin qu'ils puissent être détectés.

Pour ce faire, un procédé de sonication destiné à fragmenter les prions est mis en oeuvre. Tous les prions issus de cette fragmentation par ultra-sons, repoussent in-vitro à l'aide des protéines PrP^{C} de l'échantillon. Ce cycle élémentaire (fragmentation-repousse) est reproduit autant de fois que nécessaire, jusqu'à ce que la quantité de prions devienne détectable.

L'étape de détection, facilitée par l'étape d'amplification, est effectuée par spectroscopie de fluorescence. Les protéines à détecter sont marquées à l'aide d'une sonde fluorescente, dont la présence se manifeste lorsque celle-ci est éclairée par une lumière de longueur d'onde caractéristique. Toutefois, cette technique engendre, le plus souvent, un bruit de fond non négligeable, du fait que les sondes peuvent s'associer à d'autres molécules que les prions à détecter.

Une technique plus élaborée, dite technique de corrélation de fluorescence, met en oeuvre deux sondes fluorescentes qui émettent selon deux longueurs d'onde différentes et qui s'accrochent toutes deux symétriquement et spécifiquement sur les protéines PrP^{C}, ces dernières se trouvant en concentration importante au sein des prions. On éclaire ensuite avec des faisceaux à deux longueurs d'onde différentes, un volume très petit du liquide contenant les prions à détecter et on détecte, de manière instationnaire, les deux émissions fluorescentes intenses dues à la présence de prions, qui constituent un agglomérat de protéines PrP normales ou infectieuses sur lesquelles sont accrochées les sondes fluorescentes. Toutefois, un bruit de fond subsiste, dû notamment à la présence de sondes fluorescentes n'ayant pu trouver de sites d'adsorption spécifiques et à la présence de protéines normales PrP^{C} isolées, auxquelles se sont accrochées des sondes fluorescentes.

Ainsi, les procédés de concentration, exposés précédemment, présentent tous l'un ou plusieurs des inconvénients suivants :
- ils ne permettent pas une concentration spécifique des macromolécules ou agglomérats à concentrer, du fait que certaines de ces techniques peuvent générer des espèces, pouvant entraîner, notamment lors d'une éventuelle détection, un bruit de fond important ;
- ils ne permettent pas une concentration suffisante des macromolécules ou agglomérats en vue d'une éventuelle détection.

### EXPOSE DE L'INVENTION.

La présente invention a précisément pour objet de proposer un procédé de concentration de macrolécules ou d'agglomérats de molécules ou de particules, qui permette notamment une concentration sélective desdites macromolécules ou desdits agglomérats, en vue d'une éventuelle détection en limitant autant que possible les bruits de fond, ou en vue d'une éventuelle purification d'un échantillon contenant lesdites macromolécules ou lesdits agglomérats.

Pour ce faire, la présente invention a pour objet un procédé de concentration sélective d'une macromolécule ou d'un agglomérat de molécules ou de particules contenu dans un échantillon liquide, comprenant successivement les étapes suivantes :
- formation d'une dispersion stabilisée de type mousse ou émulsion, à partir d'un milieu comprenant ledit échantillon liquide et une couche interfaciale, ladite couche interfaciale étant apte à fixer sélectivement ladite macromolécule ou ledit agglomérat à concentrer ; et
- résorption de la dispersion formée lors de l'étape précédente de façon à reformer ladite couche interfaciale.

Par couche interfaciale, on entend, selon l'invention, une monocouche (ou zone quasi-bidimensionnelle) localisée à la surface de l'échantillon liquide (dite première phase liquide) comprenant la macromolécule ou l'agglomérat à concentrer. De part sa nature et ses propriétés spécifiques, cette couche est apte à assurer le transfert sélectif de la macromolécule ou de l'agglomérat de l'échantillon liquide vers la couche interfaciale, et, du fait de son volume infime par rapport à l'échantillon liquide, à concentrer ladite macromolécule ou ledit agglomérat.

Selon la nature de la macromolécule ou de l'agglomérat à concentrer, la couche interfaciale peut correspondre à une deuxième phase liquide déposée à la surface de l'échantillon liquide (cas par exemple, lorsqu'il s'agit de concentrer une macromolécule du type ADN), cette phase présentant des caractéristiques telles qu'elle permet d'attirer la macromolécule ou l'agglomérat vers elle. La couche interfaciale peut correspondre également à l'interface entre l'atmosphère ambiante et l'échantillon liquide (par exemple, lorsque la macromolécule ou l'agglomérat à concentrer présente un caractère hydrophobe, tel que les prions, et que l'échantillon liquide le contenant est un milieu aqueux).

Dans le cas où la couche interfaciale correspond à une deuxième phase liquide, le procédé de l'invention peut comprendre une étape préalable (située avant l'étape de formation de la dispersion) consistant à déposer à la surface de l'échantillon liquide ladite deuxième phase liquide ou couche interfaciale.

Généralement, l'étape de formation de la dispersion est effectuée par agitation mécanique du milieu comprenant l'échantillon liquide et la couche interfaciale ou par injection directement dans l'échantillon liquide surmontée de la couche interfaciale de jets capillaires gazeux ou liquides.

On note qu'une mousse désigne une dispersion comprenant un ensemble de bulles de gaz (typiquement d'air) coexistant avec un milieu liquide interstitiel, sous forme de minces films intersticiels entre les bulles. Ce type de dispersion ménage ainsi une multitude d'interfaces liquide-gaz. La mousse, selon l'invention, peut être obtenue, par exemple, par agitation mécanique vigoureuse du milieu susmentionné ou par injection au sein de ce milieu de jets capillaires gazeux (typiquement des jets d'air).

On note que, selon l'invention, une émulsion désigne une dispersion, dans laquelle la couche interfaciale est divisée en globules au sein de l'échantillon liquide, ledit échantillon liquide constituant un milieu interstitiel. On forme ainsi une multitude d'interfaces liquide-liquide, et on augmente de ce fait considérablement la surface de contact entre l'échantillon liquide et la couche interfaciale.

L'émulsion, au même titre que la mousse, peut être obtenue par agitation mécanique du milieu comprenant l'échantillon liquide et la couche interfaciale mais également par injection directe dans l'échantillon liquide surmonté de la couche interfaciale de jets capillaires liquides ou gazeux.

Ainsi, selon l'invention, le fait de passer par une dispersion du type mousse ou émulsion pour assurer la concentration d'une macromolécule ou d'un agglomérat de molécules ou particules contribue à ménager une multitude de zones interstitielles entre l'échantillon liquide et la couche interfaciale, ce qui augmente considérablement la quantité de surface entre ces deux milieux et facilite, de ce fait, la fixation des macromolécules ou les agglomérats de molécules par la couche interfaciale dispersée. Cette fixation se fait dans des zones quasi-bidimensionnelles, du fait qu'elle a lieu au niveau des zones interstitielles, ce qui améliore grandement l'efficacité et le temps de capture des macromolécules à concentrer par la couche interfaciale.

Si la structure de la couche interfaciale fonctionnalisée est potentiellement instable sous l'effet de la création d'aire interfaciale, on pourra mettre à profit l'instabilité de Rayleigh pour réaliser une émulsion dans des conditions quasi-statiques ; le liquide le plus dense se trouvant au-dessus du liquide le moins dense dans les conditions initiales.

Après résorption de la dispersion, on se retrouve, ainsi, en présence d'une couche interfaciale reformée concentrée en macrolécules ou agglomérats de molécules et d'une phase liquide correspondant à l'échantillon liquide dépourvu en tout ou partie desdites macromolécules ou desdits agglomérats.

L'avantage de la présente invention est donc de pouvoir concentrer rapidement et sans amplification, les macromolécules ou agglomérats et ceci de manière sélective, en passant par la formation d'une dispersion qui augmente l'efficacité de la concentration.

Comme décrit précédemment, après capture des macromolécules ou agglomérat de molécules ou particules à concentrer, la dispersion est amenée à subir une étape de résorption. Cette étape peut être menée de différentes manières. Par exemple, cette étape de résorption de la dispersion peut être effectuée par drainage des films interstitiels, dans le cas d'une mousse, ou du milieu interstitiel, dans le cas d'une émulsion. La cinétique de la résorption peut être contrôlée soit par un choix judicieux, le cas échéant, des molécules constitutives de la couche interfaciale des macromolécules ou agglomérats via la longueur des chaînes moléculaires, par exemple, à l'aide d'un cisaillement mécanique de la dispersion.

Selon la nature de la couche interfaciale et de la macromolécule ou de l'agglomérat à concentrer, et notamment lorsque le couche interfaciale correspond à une deuxième phase liquide, la couche interfaciale peut comprendre au moins une molécule apte à fixer sélectivement les macromolécules ou les agglomérats de molécules ou particules en question.

Selon ce mode de réalisation, la molécule apte à fixer la macromolécule ou agglomérat de molécules ou particules à concentrer est contenue dès le départ dans la couche interfaciale avant formation de la mousse ou de l'émulsion ; elle peut être une molécule comportant des groupements aptes à fixer la macromolécule ou ledit agglomérat par affinité chimique, polarisation électrique ou magnétique, et/ou ionisation, ladite molécule pouvant être, de préférence, une molécule surfactante.

Si ladite molécule n'est pas mélangée avec d'autres molécules surfactantes, ladite molécule peut être également, du fait de propriétés surfactantes, une molécule stabilisatrice de la dispersion formée au cours d'une des étapes du procédé.

Dans ce cas, la molécule assure une double fonction, qui est de fixer la macromolécule ou l'agglomérat à concentrer et également d'assurer la stabilisation de la dispersion, contribuant ainsi à augmenter le temps de contact au niveau des zones interstitielles entre molécules attractives et macromolécules ou agglomérats à concentrer.

Le procédé selon l'invention peut s'appliquer à la concentration de tout type de macromolécules ou d'agglomérats.

A titre d'exemples, on peut citer comme macromolécules pouvant être concentrées, selon le procédé de l'invention, les acides nucléiques, les protéines telles les anticorps ou les antigènes.

A titre d'exemples, on peut citer comme agglomérats de molécules pouvant être concentrés, selon le procédé de l'invention, les prions.

A titre d'exemples, on peut citer comme agglomérats de particules pouvant être concentrés des particules colloïdales telles que des particules d'or.

Ainsi, le procédé selon l'invention peut être mis en oeuvre pour la concentration d'un acide nucléique particulier, qui est l'ADN.

Dans le cas de l'ADN, la couche interfaciale correspond à une deuxième phase liquide comprenant une molécule apte à fixer l'ADN, qui est, par exemple, une molécule fonctionnalisée par une sonde (telle qu'un ADN complémentaire de l'ADN à concentrer) de façon à permettre l'hybridation spécifique de l'ADN à concentrer, par exemple, un lipide fonctionnalisé par un ADN complémentaire de l'ADN à concentrer.

Le fait que la molécule soit un lipide est particulièrement intéressant dans le cadre de cette invention, car cette catégorie de molécules contribue à assurer la stabilisation de la dispersion formée. De plus, la fonctionnalisation de cette molécule par un ADN complémentaire de l'ADN à concentrer permet une concentration et une extraction sélective dudit ADN.

A titre d'exemples, on peut citer, comme lipides fonctionnalisés efficaces dans la concentration d'ADN, des lipides biotinylés comportant un groupement avidine ou dérivé de l'avidine, sur lequel est greffé l'ADN complémentaire par une extrémité biotinylée précédemment incorporée audit ADN complémentaire ou encore des lipides cationiques comprenant au moins un groupement spermine sur lequel est adsorbé l'ADN complémentaire. De tels lipides cationiques peuvent être des lipides du type DOGS ou Dioctadécylamidoglycylspermine, commercialisé sous la marque Transfectam^{™}. Ces lipides présentent deux chaînes carbonées saturées en C₁₈ ainsi qu'une tête polaire constituée d'un groupement spermine présentant une forte affinité pour l'ADN. L'ADN complémentaire est ainsi adsorbé sur les sites spermine. Les lipides résultant constituent de véritables sondes fonctionnalisées permettant l'hydridation spécifique des ADN à concentrer, dit 'ADN cibles'.

Le procédé selon l'invention peut être utilisé pour la concentration sélective d'antigènes ou d'anticorps contenus dans un échantillon liquide, sans passer par un support solide, tel que cela est le cas des réalisations de l'art antérieur. Selon ce mode de réalisation, la couche interfaciale correspond à une deuxième phase liquide déposée à la surface de l'échantillon liquide et comprend des vésicules ou liposomes constitués de phospholipides et contenant l'anticorps ou l'antigène complémentaire de l'antigène ou l'anticorps à concentrer.

Le procédé selon l'invention peut également être utilisé pour la concentration d'agglomérats de molécules, tels que les prions. Dans le cas des prions, la couche interfaciale correspond à l'interface (échantillon liquide/atmosphère ambiante), la couche interfaciale étant apte à concentrer sélectivement les prions, du fait du caractère hydrophobe des prions (l'échantillon liquide étant un milieu aqueux tel que le sang).

Le procédé selon l'invention peut également être utilisé pour la concentration de particules colloïdales. Celles-ci peuvent être, par exemple, des particules submicroniques d'or solubilisées dans l'eau (correspondant selon la terminologie de l'invention à la première phase liquide). Dans ce cas, la couche interfaciale correspond à une deuxième phase liquide comprenant des molécules aptes à fixer ces particules colloïdales d'or, lesdites molécules étant, par exemple, des molécules portant des groupements thiol - SH.

Le procédé de concentration, explicité ci-dessus, sert, comme son nom l'indique, à concentrer sélectivement dans une couche interfaciale une macromolécule ou un agglomérat de macromolécules donné. Il peut, de ce fait, être mis en oeuvre, afin de purifier, de détecter ou d'amplifier la macromolécule ou un agglomérat de molécules ou particules donné.

Ainsi, la présente invention a également pour objet un procédé de purification d'une macromolécule ou d'un agglomérat de molécules ou particules contenu initialement dans un échantillon liquide, comprenant la concentration de ladite macromolécule ou dudit agglomérat au sein de ladite couche interfaciale par la mise en oeuvre du procédé de concentration décrit précédemment suivie de l'élimination de l'échantillon liquide appauvri en ladite macromolécule ou ledit agglomérat, après l'étape de concentration.

Par exemple, ce procédé trouve son application dans le cas de la purification d'ADN. Dans ce cas, le procédé selon l'invention consiste à partir de molécules fonctionnalisées par un ADN complémentaire spécifique, à extraire spécifiquement un ADN cible, à partir d'un échantillon liquide comprenant, par exemple, un mélange de divers ADN ou diverses portions d'ADN, ledit échantillon étant ensuite éliminé.

Ce procédé peut également trouver son application dans la purification de protéines. La capture sélective de protéines via la couche de lipides fonctionnalisés, suivi d'une étape ultérieure de cristallisation desdites protéines peut permettre d'isoler ces protéines afin d'en étudier leur structure ou bien permettre de purifier une solution de la protéine en question.

La présente invention a également pour objet un procédé de détection d'une macromolécule ou d'un agglomérat de molécules ou particules contenu initialement dans un échantillon liquide comprenant la concentration au sein d'une couche interfaciale de ladite macromolécule ou dudit agglomérérat par la mise en oeuvre du procédé de concentration décrit précédemment suivie de la détection de ladite macromolécule ou dudit agglomérat au sein de ladite couche par des techniques appropriées de détection.

Ainsi, lorsque la macromolécule est de l'ADN, la détection de l'ADN, après concentration sélective, peut se faire par fluorescence excitée par laser ou en détectant la variation du potentiel électrique de surface au niveau de la couche fonctionnalisée, ou encore par une technique de rhéologie interfaciale. Les performances de la détection par fluorescence ou électrique peuvent être notamment améliorées en comprimant par un procédé mécanique ou hydrodynamique la couche interfaciale contenant les ADN cibles hybridés en un point de l'interface (au centre, par exemple) coïncidant avec le volume laser d'excitation ou bien avec la présence d'une sonde électrique.

Enfin, la présente invention a également pour objet un procédé d'amplification d'une macromolécule ou d'un agglomérat de molécules ou particules contenu initialement dans un échantillon liquide comprenant la concentration de ladite macromolécule ou dudit agglomérat au sein d'une couche interfaciale par la mise en oeuvre du procédé de concentration décrit précédemment, le remplacement dudit échantillon liquide, après l'étape de concentration de ladite macromolécule ou dudit agglomérat au sein de ladite couche, par un liquide comprenant des agents d'amplification, suivie de l'étape d'amplification au moyen desdits agents.

Ainsi, lorsque le procédé d'amplification s'applique à l'ADN, après concentration des segments d'ADN cibles dans la couche interfaciale, l'échantillon liquide appauvri en ces segments est soutiré et remplacé par un liquide purifié contenant des agents d'amplification tels que la polymérase et des désoxyribonucléotides. La phase d'amplification par PCR peut être alors réalisée sans la présence de segments d'ADN parasites et le bruit de fond de la PCR s'en trouve considérablement réduit.

Par exemple, lorsque le procédé d'amplification s'applique à des agglomérats de molécules tels que les prions, après concentration des prions dans une couche interfaciale donnée, selon le procédé de concentration décrit précédemment, l'échantillon liquide appauvrie en lesdits prions peut être soutirée et remplacée par un liquide pur contenant des agents d'amplification tels que des protéines normales PrP^{C} non encore transformées. Ensuite, les étapes classiques de la PMCA (sonication, ...) peuvent être mises en oeuvre sans être gênées par des molécules parasites.

Ce procédé d'amplification peut être suivi d'une détection ultra-sensible, par exemple, par corrélation de fluorescence, effectuée conjointement ou non à la PMCA. Par cette technique, on peut améliorer les performances de la détection en concentrant, à l'aide d'un procédé mécanique ou hydrodynamique, les prions localement en un point de la couche interfaciale qui coïncide avec le volume de mesure laser ou bien avec la présence d'une sonde électrique.

D'autres caractéristiques et avantages de l'invention apparaîtront mieux à la lecture de la description du mode de réalisation particulier qui suit, en référence aux dessins annexés.

### BREVE DESCRIPTION DES FIGURES.

La figure 1 représente les différentes étapes, pour arriver à la concentration d'un ADN cible, selon un mode particulier de réalisation du procédé de concentration selon l'invention.

La figure 2 représente une vue détaillée d'un film interstitiel créé lors de la formation d'une mousse.

Les figures 3, 4 et 5 représentent des procédés de purification, de détection et de concentration mises en oeuvre après le procédé de concentration explicité sur la figure 1.

### EXPOSE D'UN MODE PARTICULIER DE REALISATION DE L'INVENTION.

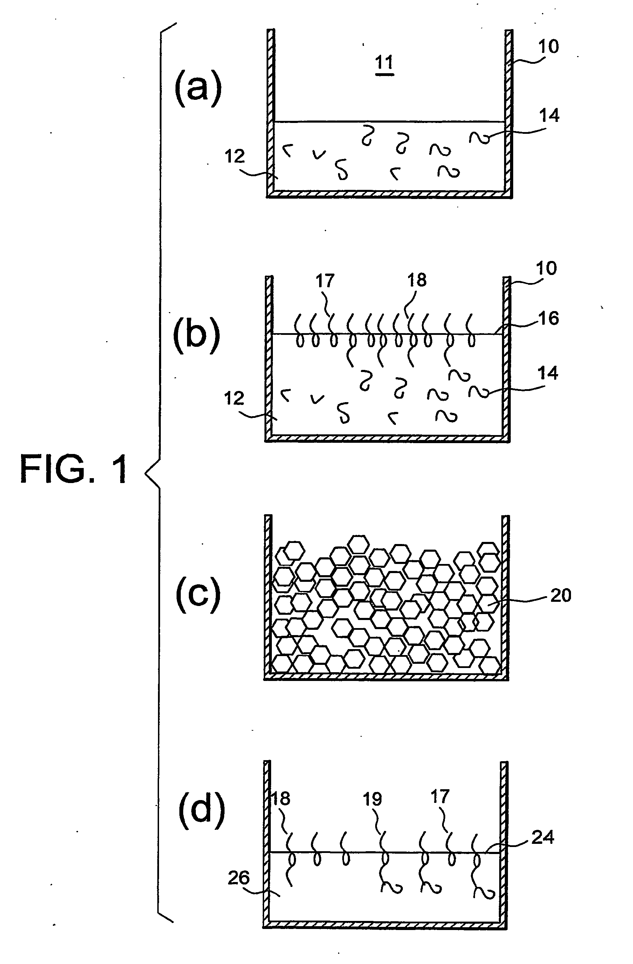

La figure 1 représente, à titre illustratif et non limitatif, la mise en oeuvre, en quatre étapes (a), (b), (c) et (d), du procédé selon l'invention pour la concentration d'un ADN cible.

L'étape (a) de la figure 1 représente un microbécher 10, dans lequel est déposé, à l'aide d'une micropipette ou d'une seringue, un échantillon liquide 12 contenant l'ADN cible 14, représenté sur la figure sous forme de brins. Ledit échantillon 12 est surmonté de l'atmosphère ambiante 11.

Au cours de l'étape intitulée (b) sur la figure 1, une monocouche 16 comprenant un mélange de lipides ligands 18 et de lipides diluants 17 (rapport lipides ligants/lipides diluants 1 :4) est déposée à la surface de l'échantillon liquide 12. Cette monocouche 16 correspond, selon la terminologie de l'invention, à une couche interfaciale. Ces lipides ligands 18 sont tels que les ADN cibles vont pouvoir s'hybrider avec lesdits lipides. Pour cela, ces lipides doivent, au cours d'une étape préliminaire, être fonctionnalisés. Ainsi, les lipides ligands peuvent être des lipides initialement biotinylés, du type biotine-(LC)-DPPE, sur lesquels on adsorbe dans un premier temps de l'avidine puis dans un deuxième temps l'on greffe l'ADN complémentaire de l'ADN cible, sur l'avidine par le biais d'un groupement biotine fixé sur l'une des extrémités de l'ADN complémentaire. L'ensemble (lipide biotinylé-Avidine-ADN complémentaire biotinylé) constitue un lipide fonctionnalisé par une sonde permettant une hybridation spécifique des brins d'ADN cible.

Les lipides peuvent être également des lipides cationiques comprenant au moins une extrémité spermine, sur laquelle est adsorbée un ADN complémentaire de l'ADN cible à concentrer.

Il est bien entendu que les molécules aptes à fixer de l'ADN cible peuvent s'étendre à tout type de molécules aptes à fixer sélectivement l'ADN cible.

Conformément à l'invention et comme l'illustre l'étape (c) de la figure 1, une dispersion 20 du type mousse est créée par injection d'air dans l'échantillon liquide, ladite mousse étant constituée d'un ensemble de bulles, maintenu en cohésion par des films liquides interstitiels. La stabilité temporaire de la mousse est assurée par les lipides constitutifs de la phase sous forme de monocouche 16.

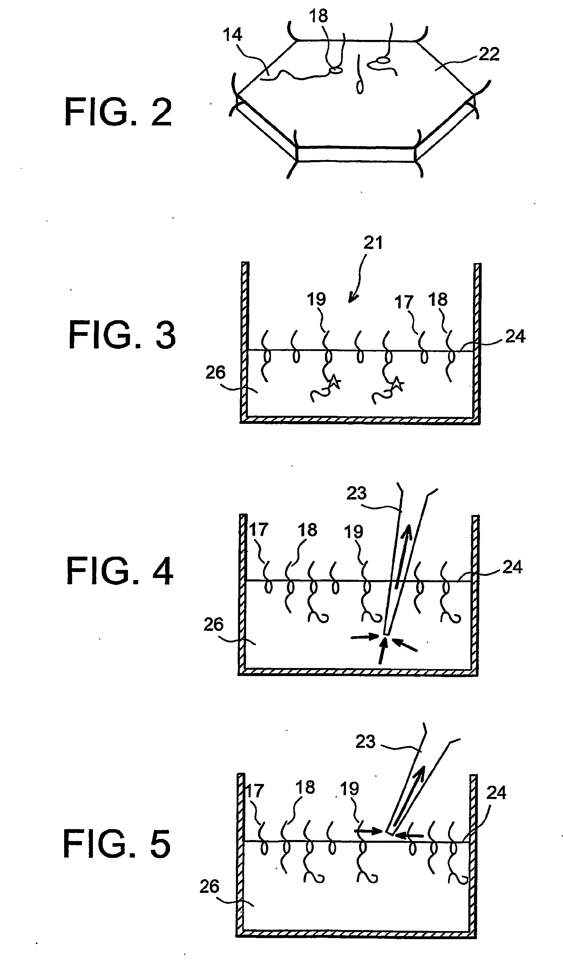

Une vue détaillée d'un film liquide interstitiel constitutif de la dispersion de type mousse est représentée sur la figure 2. Sur cette figure; ce film présente une forme octaédrique 22 de très faible volume, au sein duquel coexistent l'échantillon liquide 12 et la monocouche 16 ou couche interfaciale. De ce fait, l'ADN cible 14 se trouve quasiment en contact direct avec les lipides fonctionnalisés 18 de la monocouche ou couche interfaciale et se trouve ainsi très vite adsorbé par ces lipides 18. Le fait de passer par une mousse multiplie l'efficacité et la cinétique de capture de l'ADN par les lipides, du fait du très faible volume des films insterstitiels constitutifs de la mousse.

Enfin, au cours d'une ultime étape représentée en (d) de la figure 1, la mousse est résorbée, laissant place à nouveau à un milieu biphasique non dispersé comprenant la monocouche de lipidique 24, de très faible épaisseur, au niveau de laquelle s'est adsorbé les ADN cibles sur les lipides hybridés 19, cette monocouche correspondant à une couche interfaciale référencée 24 sur le dessin et une phase 26 correspondant à l'échantillon liquide 12 appauvri en ADN cible. Il est bien entendu, que, sur l'ensemble de la figure 1, les lipides 17, 18 et 19 ne devraient être localisés uniquement qu'au niveau de la monocouche 16 ou couche interfaciale 24 (pour la figure 1d), d'épaisseur infime. Toutefois, pour des raisons de visibilité, lesdits lipides ont été considérablement grossis.

Les figures 3, 4 et 5 illustrent différentes applications envisageables du procédé de concentration, mises en oeuvre selon un mode de réalisation particulier décrit ci-dessus.

Ainsi, la figure 3 illustre le cas où, après concentration desdits ADN cibles, par le procédé explicité sur la figure 1, lesdits ADN sont détectés par des techniques de fluorescence. Dans ce cas, les lipides 19 fonctionnalisés par un ADN complémentaire de l'ADN cible et hybridés, comportent, en plus, un marqueur fluorescent. Ainsi, on peut déterminer la présence d'ADN cible au niveau de la couche interfaciale 24 par une mesure de fluorescence.

La figure 4 illustre un cas particulier de purification d'un ADN préalablement concentré, par le procédé de concentration explicité selon la figure 1. Une fois que la résorption de la mousse est achevée, la phase 26 appauvrie en ADN, ledit ADN étant adsorbé, en majeure partie sur la couche 24, est soutirée à l'aide d'une micropipette 23 et l'on obtient une couche interfaciale 24 d'ADN purifié.

La figure 5 illustre le cas où le procédé de concentration est utilisé dans le seul but d'obtenir une phase plus concentrée en un ADN donné que la phase d'origine dudit ADN. A ce moment-là, la couche 24 riche en ADN cible est soutirée à l'aide d'une micropipette 23 pour être utilisée pour des applications diverses.

Il est bien entendu, que d'autres applications, non représentées sur ces figures, peuvent être envisagées.

## Revendications

1. Procédé de concentration sélective d'une macromolécule ou d'un agglomérat de molécules ou de particules contenu dans un échantillon liquide, comprenant successivement les étapes suivantes :
- formation d'une dispersion stabilisée de type mousse ou émulsion, à partir d'un milieu comprenant ledit échantillon liquide et une couche interfaciale, ladite couche interfaciale étant apte à fixer sélectivement ladite macromolécule ou ledit agglomérat à concentrer ; et
- résorption de la dispersion formée lors de l'étape précédente de façon à reformer ladite couche interfaciale.

2. Procédé de concentration selon la revendication 1, dans lequel l'étape de formation de la dispersion est effectuée par agitation mécanique du milieu comprenant ledit échantillon liquide et ladite couche interfaciale.

3. Procédé de concentration selon la revendication 1, dans lequel l'étape de formation de la dispersion est effectuée par injection directement dans l'échantillon liquide de jets capillaires gazeux ou liquides.

4. Procédé de concentration selon l'une quelconque des revendications 1 à 3, dans lequel la couche interfaciale comprend au moins une molécule apte à fixer sélectivement ladite macromolécule ou ledit agglomérat.

5. Procédé de concentration selon la revendication 4, dans lequel la molécule apte à fixer la macromolécule ou agglomérat de molécules ou de particules à concentrer est une molécule comportant des groupements aptes à fixer la macromolécule ou agglomérat par affinité chimique, polarisation électrique ou magnétique, et/ou ionisation, ladite molécule étant, de préférence, une molécule surfactante.

6. Procédé de concentration selon l'une quelconque des revendications 1 à 5, dans lequel la macromolécule est choisie parmi le groupe constitué par les acides nucléiques et les protéines, telles que les antigènes et les anticorps.

7. Procédé de concentration selon l'une quelconque des revendications 1 à 3, dans lequel l'agglomérat de molécules est un prion.

8. Procédé de concentration selon l'une quelconque des revendications 1 à 5, dans lequel l'agglomérat de particules est choisi parmi le groupe constitué de particules colloïdales.

9. Procédé selon l'une quelconque des revendications 1 à 6, dans lequel la macromolécule à concentrer est l'ADN.

10. Procédé selon la revendication 4, dans lequel, lorsque la macromolécule à concentrer est l'ADN, la molécule apte à fixer l'ADN est fonctionnalisée par une sonde de façon à permettre l'hybridation spécifique de l'ADN à concentrer.

11. Procédé selon la revendication 10, dans lequel la molécule apte à fixer l'ADN est un lipide fonctionnalisé par une sonde ADN complémentaire de l'ADN à concentrer.

12. Procédé selon la revendication 11, dans lequel le lipide est un lipide biotinylé comportant un groupement avidine ou dérivé de l'avidine, sur lequel est greffé l'ADN complémentaire par une extrémité biotinylée précédemment incorporée audit ADN.

13. Procédé selon la revendication 11, dans lequel le lipide est un lipide cationique comprenant au moins un groupement spermine sur lequel est adsorbé l'ADN complémentaire.

14. Procédé de purification d'une macromolécule ou d'un agglomérat de molécules ou particules contenu initialement dans un échantillon liquide, comprenant la concentration de ladite macromolécule ou dudit agglomérat au sein d'une couche par la mise en oeuvre du procédé selon l'une quelconque des revendications 1 à 13, l'élimination de l'échantillon liquide appauvri en ladite macromolécule ou ledit agglomérat, après l'étape de concentration.

15. Procédé de détection d'une macromolécule ou d'un agglomérat de molécules ou particules contenu initialement dans un échantillon liquide comprenant la concentration au sein d'une couche de ladite macromolécule ou dudit agglomérat par la mise en oeuvre du procédé selon l'une quelconque des revendications 1 à 13 et la détection de ladite macromolécule ou dudit agglomérat au sein de ladite couche par des techniques appropriées de détection.

16. Procédé d'amplification d'une macromolécule ou d'un agglomérat de molécules ou particules contenu initialement dans un échantillon liquide comprenant la concentration de ladite macromolécule ou dudit agglomérat au sein d'une couche par la mise en oeuvre du procédé selon l'une quelconque des revendications 1 à 13 et le remplacement dudit échantillon liquide, après l'étape de concentration de ladite macromolécule ou dudit agglomérat au sein de ladite couche, par un liquide comprenant des agents d'amplification, suivie de l'étape d'amplification au moyen desdits agents.

17. Procédé d'amplification selon la revendication 16, dans lequel la macromolécule est un ADN.

18. Procédé d'amplification selon la revendication 16, dans lequel l'agglomérat de molécules est un prion.

## Claims

1. Method for the selective concentration of a macromolecule or of an agglomerate of molecules or of particles contained in a liquid sample, comprising successively the following steps:
- formation of a stabilized dispersion of foam or emulsion type, from a medium comprising said liquid sample and an interface layer, said interface layer being capable of selectively fixing said macromolecule or said agglomerate to be concentrated; and
- resorption of the dispersion formed during the preceding step so as to reform said interface layer.

2. Concentration method according to Claim 1, in which the dispersion formation step is carried out by mechanical agitation of the medium comprising said liquid sample and said interface layer.

3. Concentration method according to Claim 1, in which the dispersion formation step is carried out by injection, directly into the liquid sample, of gaseous or liquid capillary jets.

4. Concentration method according to any one of Claims 1 to 3, in which the interface layer comprises at least one molecule capable of selectively fixing said macromolecule or said agglomerate.

5. Concentration method according to Claim 4, in which the molecule capable of fixing the macromolecule or agglomerate of molecules or of particles to be concentrated is a molecule comprising groups capable of fixing the macromolecule or agglomerate by chemical affinity, electric or magnetic polarization, and/or ionization, said molecule preferably being a surfactant molecule.

6. Concentration method according to any one of Claims 1 to 5, in which the macromolecule is chosen from the group consisting of nucleic acids and proteins, such as antigens and antibodies.

7. Concentration method according to any one of Claims 1 to 3, in which the agglomerate of molecules is a prion.

8. Concentration method according to any one of Claims 1 to 5, in which the agglomerate of particles is chosen from the group consisting of colloidal particles.

9. Method according to any one of Claims 1 to 6, in which the macromolecule to be concentrated is DNA.

10. Method according to Claim 4, in which, when the macromolecule to be concentrated is DNA, the molecule capable of fixing the DNA is functionalized with a probe so as to allow the specific hybridization of the DNA to be concentrated.

11. Method according to Claim 10, in which the molecule capable of fixing the DNA is a lipid functionalized with a DNA probe complementary to the DNA to be concentrated.

12. Method according to Claim 11, in which the lipid is a biotinylated lipid comprising an avidin group or avidin derivative, onto which the complementary DNA is grafted by means of a biotinylated end incorporated into said DNA beforehand.

13. Method according to Claim 11, in which the lipid is a cationic lipid comprising at least one spermine group onto which the complementary DNA is adsorbed.

14. Method for the purification of a macromolecule or of an agglomerate of molecules or particles initially contained in a liquid sample, comprising the concentration of said macromolecule or of said agglomerate within a layer using the method according to any one of Claims 1 to 13, and the elimination of the liquid sample depleted of said macromolecule or said agglomerate, after the concentration step.

15. Method for the detection of a macromolecule or of an agglomerate of molecules or particles initially contained in a liquid sample, comprising the concentration, within a layer, of said macromolecule or of said agglomerate using the method according to any one of Claims 1 to 13, and the detection of said macromolecule or of said agglomerate within said layer by means of appropriate detection techniques.

16. Method for the amplification of a macromolecule or of an agglomerate of molecules or of particles initially contained in a liquid sample, comprising the concentration of said macromolecule or of said agglomerate within a layer using the method according to any one of Claims 1 to 13, and the replacement of said liquid sample, after the step for concentrating said macromolecule or said agglomerate, within said layer, with a liquid comprising amplification agents, followed by the step of amplification by means of said agents.

17. Amplification method according to Claim 16, in which the macromolecule is a DNA.

18. Amplification method according to Claim 16, in which the agglomerate of molecules is a prion.

## Patentansprüche

1. Verfahren zum selektiven Aufkonzentrieren eines Makromoleküls oder eines Agglomerats von Molekülen oder Teilchen, das in einer flüssigen Probe enthalten ist, wobei das Verfahren die folgenden Stufen umfasst:
- Herstellung einer stabilisierten Dispersion vom Schaum- oder Emulsions-Typ aus einem Medium, das die genannte flüssige Probe und eine Grenzflächenschicht enthält, die in der Lage ist, das genannte Makromolekül oder das genannte Agglomerat, das aufkonzentriert werden soll, selektiv zu fixieren, und
- Resorption der in der vorhergehenden Stufe gebildeten Dispersion zur Rückbildung der genannten Grenzflächenschicht.

2. Verfahren zum Aufkonzentrieren nach Anspruch 1, in dem die Stufe der Bildung der Dispersion durchgeführt wird durch mechanisches Rühren des Mediums, das die genannte flüssige Probe und die genannte Grenzflächenschicht enthält.

3. Verfahren zum Aufkonzentrieren nach Anspruch 1, bei dem die Stufe der Bildung der Dispersion durchgeführt wird durch direkte Injektion von Gas- oder Flüssigkeits-Kapillarstrahlen in die flüssige Probe.

4. Verfahren zum Aufkonzentrieren nach einem der Ansprüche 1 bis 3, bei dem die Grenzflächenschicht mindestens ein Molekül umfasst, welches das genannte Makromolekül oder das genannte Agglomerat selektiv fixieren kann.

5. Verfahren zum Aufkonzentrieren nach Anspruch 4, bei dem das Molekül, welches das Makromolekül oder das Agglomerat von Molekülen oder Teilchen, das aufkonzentriert werden soll, fixieren kann, ein Molekül ist, das Gruppen umfasst, die das Makromolekül oder das Agglomerat durch chemische Affinität, durch elektrische oder magnetische Polarisation und/oder durch Ionisation fixieren können, wobei das genannte Molekül vorzugsweise ein grenzflächenaktives Molekül ist.

6. Verfahren zum Aufkonzentrieren nach einem der Ansprüche 1 bis 5, bei dem das Makromolekül ausgewählt wird aus der Gruppe, die besteht aus Nucleinsäuren und Proteinen, beispielsweise aus Antigenen und Antikörpern.

7. Verfahren zum Aufkonzentrieren nach einem der Ansprüche 1 bis 3, bei dem das Agglomerat von Molekülen ein Prion ist.

8. Verfahren zum Aufkonzentrieren nach einem der Ansprüche 1 bis 5, bei dem das Agglomerat von Teilchen ausgewählt wird aus der Gruppe, die besteht aus kolloidalen Teilchen.

9. Verfahren nach einem der Ansprüche 1 bis 6, bei dem das Makromolekül, das aufkonzentriert werden soll, DNA ist.

10. Verfahren nach Anspruch 4, bei dem dann, wenn das Makromolekül, das aufkonzentriert werden soll, DNA ist, das Molekül, das DNA fixieren kann, mittels einer Sonde so funktionalisiert ist, dass es die spezifische Hybridisierung der DNA, die aufkonzentriert werden soll, erlaubt.

11. Verfahren nach Anspruch 10, bei dem das Molekül, das die DNA fixieren kann, ein Lipid ist, das durch eine DNA-Sonde funktionalisiert ist, die komplementär zu der DNA ist, die aufkonzentriert werden soll.

12. Verfahren nach Anspruch 11, bei dem das Lipid ein biotinyliertes Lipid ist, das eine Avidin-Gruppe oder eine von Avidin abgeleitete Gruppe enthält, auf das die komplementäre DNA aufgepfropft ist mittels eines biotinylierten Endes, das vorher der DNA einverleibt worden ist.

13. Verfahren nach Anspruch 11, bei dem das Lipid ein kationisches Lipid ist, das mindestens eine Spermin-Gruppe aufweist, an dem die komplementäre DNA adsorbiert ist.

14. Verfahren zum Reinigen eines Makromoleküls oder eines Agglomerats von Molekülen oder Teilchen, das anfänglich in einer flüssigen Probe enthalten ist, wobei das Verfahren umfasst das Aufkonzentrieren des genannten Makromoleküls oder des genannten Agglomerats im Innern einer Schicht durch Durchführung des Verfahrens nach einem der Ansprüche 1 bis 13, und das Eliminieren der an dem genannten Makromolekül oder dem genannten Agglomerat verarmten flüssigen Probe nach dem Aufkonzentrieren.

15. Verfahren zum Nachweis eines Makromoleküls oder eines Agglomerats von Molekülen oder Teilchen, das anfänglich in einer flüssigen Probe enthalten ist, wobei das Verfahren umfasst das Aufkonzentrieren im Innern einer Schicht aus dem genannten Makromolekül oder dem genannten Agglomerat durch Durchführung des Verfahrens nach einem der Ansprüche 1 bis 13 und den Nachweis des genannten Makromoleküls oder des genannten Agglomerats im Innern der genannten Schicht durch geeignete Nachweismethoden.

16. Verfahren zur Amplifikation (Verstärkung) eines Makromoleküls oder eines Agglomerats von Molekülen oder Teilchen, das anfänglich in einer flüssigen Probe enthalten ist, wobei das Verfahren umfasst das Aufkonzentrieren des genannten Makromoleküls oder des genannten Agglomerats im Innern einer Schicht durch Durchführung des Verfahrens nach einem der Ansprüche 1 bis 13 und den Ersatz der genannten flüssigen Probe nach dem Aufkonzentrieren des genannten Makromoleküls oder des genannten Agglomerats im Innern der genannten Schicht durch eine Flüssigkeit, die Amplifikationsmittel (Verstärkungsmittel) umfasst, gefolgt von einer Stufe zur Amplifikation (Verstärkung) mittels der genannten Amplifikationsmittel.

17. Verfahren zur Amplifikation nach Anspruch 16, bei dem das Makromolekül DNA ist.

18. Verfahren zur Amplifikation nach Anspruch 16, bei dem das Agglomerat von Molekülen ein Prion ist.
